# EUROPÄISCHE PATENTSCHRIFT

(11) **EP 3 313 262 B1**
(45) Veröffentlichungstag und Bekanntmachung des Hinweises auf die Patenterteilung: **13.09.2023**
(21) Anmeldenummer: 16733939.9
(22) Anmeldetag: 28.06.2016
(51) Int. Cl.: A61B 3/10, A61B 3/113

(54) **PURKINJEMETER UND VERFAHREN ZUR AUTOMATISCHEN AUSWERTUNG**
PURKINJE METER AND METHOD FOR AUTOMATIC EVALUATION
PURKINJE-MÈTRE ET PROCÉDÉ D'ÉVALUATION AUTOMATIQUE

(30) Priorität: 29.06.2015 DE 102015110456
(43) Veröffentlichungstag der Anmeldung: 02.05.2018
(73) Patentinhaber: JANUNTS HOLDING UG, 66123 Saarbrücken (DE)
(72) Erfinder: JANUNTS, Edgar, 66123 Saarbrücken (DE); PUTZU, Dominic, 66787 Wadgassen (DE); KESSELHEIM-VAN BELLEN, Janine, 2500 Valby, Copenhagen (DK); MÖLLER, Michael, 66121 Saarbrücken (DE); LANGENBUCHER, Achim, 66424 Kirrberg (DE)
(74) Vertreter: Lecomte & Partners
(86) Internationale Anmeldenummer: PCT/EP2016/065054
(87) Internationale Veröffentlichungsnummer: WO 2017/001426

(56) Entgegenhaltungen:
- EP-A1- 2 604 180
- WO-A1-2014/073645
- JP-A- 2014 052 813
- JUAN TABERNERO ET AL: "Instrument for measuring the misalignments of ocular surfaces", OPTICS EXPRESS, Bd. 14, Nr. 22, 30. Oktober 2006 (2006-10-30), Seite 10945, XP055303879, ISSN: 2161-2072, DOI: 10.1364/OE.14.010945 in der Anmeldung erwähnt

## Beschreibung

### Gebiet der Erfindung

Die Erfindung betrifft das Gebiet der Bilderfassung und Bilderkennung im Bereich der ophthalmologischen Diagnostik.

### Stand der Technik

Als grauer Star oder Katarakt bezeichnet man eine Trübung der Augenlinse. Eine trübe natürliche Augenlinse kann operativ entfernt und durch ein Linsenimplantat, auch intraokulare Linse, IOL, genannt, ersetzt werden. Die Sehkraft des so behandelten Auges hängt von der Qualität und Geometrie der implantierten Linse, aber auch von deren Stellung oder Position im Auge ab.

Eine Dezentrierung oder Verkippung der Linse kann zu wesentlichen Beeinträchtigungen des Sehvermögens nach einem chirurgischen Eingriff führen. Dies ist umso relevanter wenn die implantierte Linse eine dem Auge spezifisch angepasste Geometrie aufweist. Für asphärische Linsenimplantate sind beispielsweise kritische Verkippungswerte von +/- 7° und Dezentrierungswerte von +/- 0.4 mm bekannt.

Deshalb ist es wichtig die Position einer intraokularen Linse möglichst einfach und genau erfassen zu können, um korrekt auf die Ursache einer solchen Beeinträchtigung schließen zu können. Bekannte Verfahren zum Bestimmen der Position in einem Auge umfassen optische Kohärenztomographie ("optical coherence tomography", OCT), Scheimpflug Verfahren, Ultraschall-Biomikroskopie, und Purkinjemetrie.

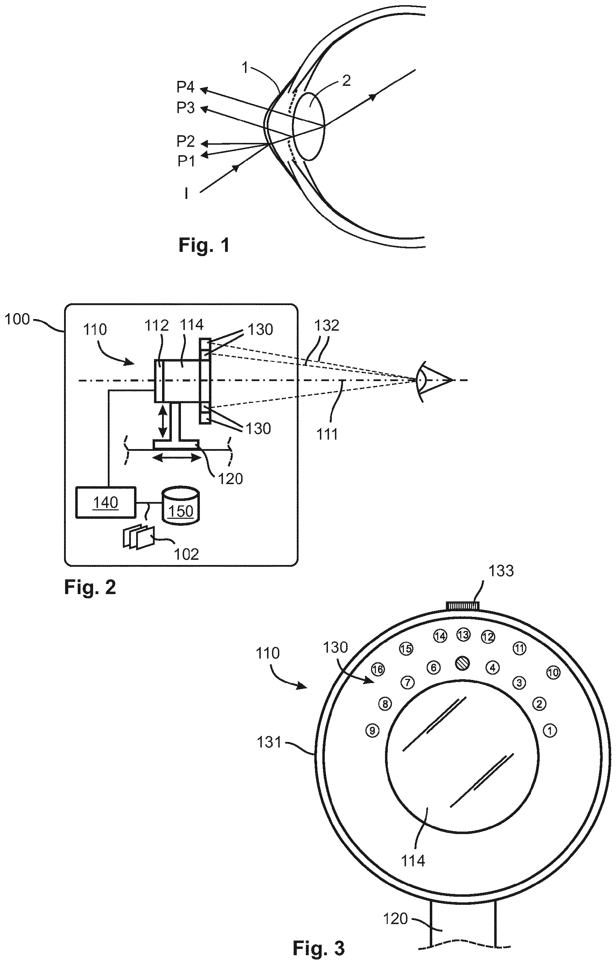

Die Purkinjemetrie beruht auf der Erkenntnis, dass Lichtstrahlen, welche in das Auge einfallen, an verschiedenen Grenzflächen unter verschiedenen Winkeln reflektiert werden. Dadurch werden die Purkinje Bilder sichtbar. Es handelt sich um die Bilder, die durch das Reflektieren der Lichtstrahlen an den verschiedenen Refraktionsflächen der Hornhaut und des Kristallkörpers oder der Linse entstehen. Anhand von Figur 1 werden die Purkinje-Reflexe näher erläutert. Das erste Bild, P1, ist die Reflektion, die an der vorderen Hornhautfläche der Hornhaut 1 entsteht. P2 entsteht an der hinteren Hornhautfläche und überlagert sich meistens mit P1, so dass P2 für weitere Berechnungen in der Regel irrelevant ist. Das dritte Bild, P3, entsteht an der vorderen Linsenfläche der Linse 2, und P4 entsteht schließlich an der hinteren Linsenfläche.

Es ist bekannt, dass durch die relative Stellung der Purkinje-Bilder zueinander Größen wie z.B. die Distanz zwischen der Linse und der Iris oder die Krümmung der Linse berechnet werden können.

Nach bestem Wissen der Anmelder sind zurzeit zwei Purkinjemeter, das heißt, Vorrichtungen zum Erfassen und Auswerten der Purkinje-Reflexe bekannt. Ein erstes und gattungsgemäßes Purkinjemeter wurde von Tabernero und Artal offenbart (Tabernero et al., Instrument for measuring the misalignment of ocular surfaces, Opt Express, 2006; 14:10945-10956) Es wird hier bekanntermaßen jeweils eine Vielzahl von halbkreisförmig angeordneten Lichtquellen zur Beleuchtung des Auges in verschiedenen Positionen genutzt. Die entsprechenden Purkinje-Reflexe P1, P3 und P4 entsprechen in der Regel nicht der Form der projizierten halbkreisförmigen Muster, da diese durch individuelle Hornhaut- und Linsengeometrien verformt werden. Eine Mustererkennung durch Annahme einer vordefinierten Form ist demnach mit einem systematischen Fehler behaftet.

Ein zweites Purkinjemeter wurde von F. Schaeffel entwickelt (Schaeffel, Binocular lens tilt and decentration measurements in healthy subjects with phakic eyes, Invest Ophtalmol Vis Sci 2008;49:2216-2222). Das Verfahren zum Erfassen der Purkinje-Reflexe dauert hier in der Regel ungefähr 10 Minuten. Es wird hier eine einzige Lichtquelle zum Beleuchten des Auges genutzt. Die untersuchte Person wird aufgefordert, verschiedene vorbestimmte Referenzpunkte anzustarren um die entsprechenden verschiedenen Positionen der Reflexe P1, P3, P4 durch manuelles Auslösen einer Kamera zu erfassen. Die erfassten Reflexe werden dann manuell nach Kenntnis der untersuchenden Person den Purkinje-Reflexen P1, P3 und P4 zugeordnet. Jeder Fehler, der dem Untersuchten oder dem Untersuchenden während des Verfahrens unterläuft, hat zur Folge, dass das Verfahren wieder neu begonnen werden muss. Somit ist dieses Purkinjemeter zumindest bei nicht-kooperativen Patienten schwer einsetzbar.

Beide der bekannten Geräte bzw. die zugrundeliegenden Methoden beruhen darauf, dass der zu untersuchende Patient für jede Einzelmessung (eine Untersuchung besteht aus einer Serie von manuellen Einzelmessungen) unterschiedliche Punkte in unterschiedlichen Blickrichtungen fokussieren muss. Somit hängt die Qualität des Messergebnisses entscheidend von der Kooperationsbereitschaft des Patienten sowie der Exaktheit/Stabilität seiner Fokussierung ab. Die Fehleranfälligkeit steigt mit der Dauer der Untersuchung, welche mehrere Minuten betragen kann. Ausserdem wird die Bildanalyse manuell ausgeführt, wobei das Zuordnen abgebildeter Reflexe zu den Purkinje-Reflexen von der Erfahrung des Bildanalysten abhängt.

Aufgabe der Erfindung ist es daher, ein Purkinjemeter und ein entsprechendes Verfahren bereit zu stellen, welches wenigstens einen der in dieser Anmeldung genannten Nachteile überwindet.

### Offenbarung der Erfindung

Die gestellte technische Aufgabe wird durch die Merkmale der erfindungsgemäßen Vorrichtung gemäß Anspruch 1 und des erfindungsgemäßen Verfahrens gemäß Anspruch 8 gelöst. Die Erfindung betrifft eine Vorrichtung zum automatischen Erkennen der Stellung einer intraokularen Linse oder einer natürlichen Linse in einem Auge. Die Vorrichtung umfasst:
- eine Kamera, welche bildgebende Mittel und eine Kameralinse umfasst, und eine Kameraachse definiert;
- Stellungsmittel, die dazu ausgebildet sind die Kamera gegenüber dem abzubildenden Auge in eine fixe, vorbestimmte Stellung zu bringen;
- eine Vielzahl an einzelnen Lichtquellen, welche so angeordnet sind, dass jede der Lichtquellen Lichtstrahlen in einem jeweils vorbestimmten Winkel gegenüber der Kameraachse und in Richtung des abzubildenden Auges abgegeben kann;
- Steuerungsmittel, die dazu ausgebildet sind mittels der Kamera, bei gleichbleibender Stellung der Kamera und der Lichtquellen, eine Bildfolge des abzubildenden Auges zu erfassen und auf einem Speichermedium zu sichern.

Die Steuerungsmittel sind dazu ausgebildet, die Lichtquellen so ein- und auszuschalten, dass während des Erfassens der Bilder die Lichtquellen in einer vorbestimmten Reihenfolge eingeschaltet werden, und dass während des Erfassens jedes der Bilder jeweils nur eine vorbestimmte Lichtquelle eingeschaltet ist, und die Vorrichtung Bearbeitungsmittel umfasst, welche dazu eingerichtet sind, auf den Bildern der Bildfolge einzelne abgebildete Lichtreflexe und deren Verschiebung entlang der Bildfolge zu erkennen, und aufgrund der erkannten Form und Verschiebung der entsprechenden Lichtreflexe spezifische Purkinje-Reflexe zuzuordnen, und aufgrund der zugeordneten spezifischen Purkinje-Reflexe auf die Stellung der intraokularen oder der natürlichen Linse in dem abgebildeten Auge zu schließen.

Bevorzugt können die Lichtquellen um die Kameralinse entlang wenigstens einem Kreisteil angeordnet sein. Vorteilhafterweise können die Lichtquellen in zwei konzentrischen, symmetrisch gegenüber einer rechtwinklig zur Kameraachse, zentral durch die Kameralinse verlaufende Achse angeordnet sein.

Die Lichtquellen können bevorzugt an einer schräg zur Kameraachse orientierten Ringfläche oder Teilringfläche angeordnet sein.

Die Lichtquellen können bevorzugt so angeordnet sein, dass abgegebene Lichtstrahlen mit hoher Wahrscheinlichkeit die Hornhaut des abzubildenden Auges rechtwinklig treffen können.

Die Vielzahl an Lichtquelle kann zum Beispiel fünfzehn Lichtquellen umfassen.

Bevorzugt können die Lichtquellen Infrarot-Leuchtdioden sein. Die bildgebenden Mittel können bevorzugter Weise infrarot-empfindliche bildgebende Mittel sein.

Die Lichtquellen können bevorzugt auf einer Trägerplatte angeordnet sein.

Die Trägerplatte kann des Weiteren einen einzigen visuell erkennbaren Referenzpunkt umfassen.

Bevorzugt kann die Trägerplatte eine zusätzliche Lichtquelle umfassen, welche sichtbares Licht abgegeben kann, und welche dazu eingerichtet ist während des Erfassens jedes der Bilder eingeschaltet zu sein.

Alternativ dazu kann die Vorrichtung ausserhalb der Trägerplatte an einer vorbestimmten Position einen einzigen visuell erkennbaren Referenzpunkt umfassen. Der Referenzpunkt kann eine zusätzliche Lichtquelle sein, welche sichtbares Licht abgeben kann, und welche dazu eingerichtet ist, während des Erfassens jedes der Bilder eingeschaltet zu sein. Das sichtbare Licht, welches von dieser Referenzlichtquelle ausgestrahlt wird, kann vorzugsweise über optische Mittel in das Sichtfeld des abzubildenden Auges eingeleitet werden. Die optischen Mittel umfassen beispielsweise wenigstens einen Spiegel.

Die Erfindung betrifft auch ein Verfahren zum automatischen Erkennen der Stellung einer intraokularen Linse oder einer natürlichen Linse in einem Auge anhand einer mittels der Vorrichtung gemäß der Erfindung erfassten Bildfolge, welche das Auge abbildet.

Das Verfahren umfasst folgende Schritte:
- Erkennen von einzelnen abgebildeten Lichtreflexen und deren Verschiebung entlang der Bildfolge;
- Zuordnen der Lichtreflexe zu Purkinje-Reflexe, mittels der erkannten Form und der Verschiebung der entsprechenden Lichtreflexe, sowie des vorbestimmten Winkels der jeweilig entsprechenden Lichtquelle, welche während des Erfassens eines jeweiligen Bildes eingeschaltet war;
- Errechnen der Dezentrierung und/oder Verkippung der Linse anhand der zugeordneten Purkinje-Reflexe.

Bevorzugt wird des Weiteren ein Umriss der Pupille und/oder der Iris des abgebildeten Auges in der Bildfolge automatisch erkannt.

Bevorzugt starrt das in der Bildfolge abgebildete Subjekt während der Bilderfassung einen einzelnen Referenzpunkt an, welcher vorzugsweise Teil der Vorrichtung ist.

Sämtliche Merkmale der oben beschriebenen Ausführungsformen können miteinander kombiniert oder gegeneinander ausgetauscht werden.

### Kurze Beschreibung der Figuren

Im Folgenden werden kurz die Figuren der Ausführungsbeispiele beschrieben. Weitere Details sind der detaillierten Beschreibung der Ausführungsbeispiele zu entnehmen. Es zeigen:
- Figur 1: zeigt eine schematische Abbildung eines Auges und illustriert das bekannte Entstehen der Purkinje-Reflexe P1, P2, P3 und P4;
- Figur 2: zeigt eine schematische Seitenansicht einer erfindungsgemäßen Vorrichtung in einer bevorzugten Ausführungsform;
- Figur 3: zeigt ein Detail einer bevorzugten Ausführungsform der erfindungsgemäßen Vorrichtung;
- Figur 4A: ist eine schematische Darstellung eines Bildes (oben), welches ein Auge abbildet, das anhand der erfindungsgemäßen Vorrichtung in einer bevorzugten Ausführungsform erfasst wurde; unten ist die entsprechende Beleuchtung abgebildet;
- Figur 4B: ist eine schematische Darstellung eines Bildes (oben) welches ein Auge abbildet, das anhand der erfindungsgemäßen Vorrichtung in einer bevorzugten Ausführungsform erfasst wurde; unten ist die entsprechende Beleuchtung abgebildet.

### Detaillierte Beschreibung der Ausführungsbeispiele

Figur 1 wurde bereits im Stand der Technik beschrieben.

Verschiedene Merkmale werden zur Klarheit isoliert oder in Kombination mit anderen Merkmalen im Rahmen eines spezifischen Ausführungsbeispiels offenbart. Allerdings sind die isolierten Merkmale einer Kombination, sowie die Kombinationen von als isoliert dargestellter Merkmale, ebenfalls als offenbart zu betrachten, es sei denn es wird klar beschrieben dass dies nicht der Fall ist.

Die Beschreibung ist nicht als die Erfindung einschränkend zu verstehen und beschränkt sich auf Merkmale die zum wesentlichen Verständnis der Erfindung beitragen. Bekannte Maßnahmen, wie zum Beispiel die Stromversorgung von Lichtquellen, oder die Datenverbindungen zwischen verschiedenen elektronischen Bauteilen der Vorrichtung, werden nicht weiter beschrieben.

Figur 2 zeigt ein bevorzugtes Ausführungsbeispiel einer erfindungsgemäßen Vorrichtung 100. Die Vorrichtung dient zum Abbilden eines Auges in einer Bildfolge. Hierzu kommt eine Kamera 110 zum Einsatz, welche bildgebende Mittel 112 und eine Kameralinse 114 umfasst. Die Kameralinse definiert das Blickfeld, welches anhand der bildgebenden Mittel 112 abgebildet werden kann. Die bildgebenden Mittel umfassen vorzugsweise eine Matrix aus CMOS-Sensoren, welche einfallendes Licht in digitale Signale umwandeln können.

Die Vorrichtung umfasst Stellungsmittel 120, die dazu ausgebildet sind die Kamera 110 gegenüber dem abzubildenden Auge in eine fixe, vorbestimmte Stellung zu bringen. In Figur 2 sind die Stellungsmittel 120 durch ein Stativ dargestellt, welches es erlaubt die Höhe der Kamera gegenüber einer horizontalen Ebene, z.B. einem Tisch, zu verändern, und/oder die Distanz gegenüber dem abzubildenden Auge zu verändern, um dieses in die fokale Ebene der Kamera zu bringen.

Die Vorrichtung kann vorzugsweise nicht dargestellte Mittel zum Festlegen der Position der zu untersuchenden Person haben, z.B. in Form einer Kinn- und/oder Stirnhalterung. Alternativ zu den abgebildeten Mitteln 120 kann ebenfalls die zu untersuchende Person gegenüber der Kamera beweglich sein.

Um das Verfahren sinnvoll ausführen zu können, wird bevorzugt dass die Pupillenachse oder die Sichtlinie ("line of sight") des Auges im Wesentlichen mit der Kameraachse 111 übereinstimmt.

Eine Vielzahl von einzelnen Lichtquellen 130 ist so angeordnet, dass jede der Lichtquellen Lichtstrahlen in einem jeweils vorbestimmten Winkel gegenüber der Kameraachse 111, bzw. der Pupillenachse oder der Sichtlinie, und in Richtung des abzubildenden Auges abgegeben kann. Die Vorrichtung umfasst vorzugsweise ein nicht dargestelltes Speicherelement, welches eine einzigartige Kennnummer jeder der Lichtquellen mit deren vorbestimmten Winkel gegenüber der Kameraachse 111, bzw. der Pupillenachse oder der Sichtlinie, in Verbindung bringt. Diese Daten können beispielsweise in einer Tabelle oder einer Datenbank abgespeichert sein. Die Lichtquellen sind vorzugsweise Leuchtdioden, LED, und senden vorzugsweise Lichtstrahlen im infrarot Bereich, also im nicht sichtbaren Teil des Lichtspektrums aus. Dies hat den Vorteil dass ein auf die Pupille treffender Lichtstrahl diese nicht dazu bringt sich zu verengen. Selbstverständlich sind die bildgebenden Mittel 112 in ihrer Lichtempfindlichkeit dem Spektrum der Lichtquellen angepasst.

Es werden vorzugsweise einzelne LEDs mit kleinem Abstrahlwinkel verwendet. Dadurch sind die Positionen der Purkinje-Reflexe klar definiert, sodass die Schwerpunkte der Lichtreflexe besser zu erkennen sind und die Wahrscheinlichkeit einer Überlappung verschiedener Reflexe reduziert ist. Dies vereinfacht die automatische Positionserkennung der Purkinje-Reflexe. Außerdem kann durch Verwendung einzelner LEDs mit geringem Abstrahlwinkel die Intensität der LED klein gehalten, und somit der Energieeintrag in das Auge reduziert werden.

Die Lichtquellen können auf irgendeine vorbestimmte Weise fix gegenüber der Kameraachse angeordnet sein, z.B. entlang einer Linie, die innerhalb einer normal zur Kamerachse orientierten Ebene liegt und die vorzugsweise kreisförmig konzentrisch um die Kameralinse verläuft.. Der entsprechende Sektor ist in dem Fall vorzugsweise derart eingeschränkt, dass die durch das ins Auge einfallende Lichtstrahlen entstehenden Purkinje-Reflexe mit hoher Wahrscheinlichkeit innerhalb der Pupillenfläche erscheinen. Bei Lichteinfall unter größeren Winkeln besteht die Gefahr, dass nicht alle drei Purkinje-Reflexe in den Sichtbereich der Iris fallen, was eine Positionsbestimmung bzw. Auswertung erschwert.

Die Vorrichtung 100 umfasst Steuerungsmittel 140, z.B. einen programmierten Computer Prozessor, die dazu ausgebildet sind mittels der Kamera 110, bei gleichbleibender Stellung der Kamera 110 und der Lichtquellen 130, eine Bildfolge 102 des abzubildenden Auges zu erfassen und auf einem Speichermedium 150 zu sichern. Solche Mittel sind an sich bekannt und ein entsprechendes Computerprogramm kann ohne erfinderische Tätigkeit anhand dieser Beschreibung von einem Fachmann geschrieben werden. Die Steuerungsmittel 140 sind programmiert, um die Lichtquellen 130 so ein- und auszuschalten, dass während des Erfassens der Bilder die Lichtquellen in einer vorbestimmten Reihenfolge eingeschaltet werden, und dass während des Erfassens jedes der Bilder jeweils nur eine vorbestimmte Lichtquelle 130 eingeschaltet ist. Vorzugsweise ist das Auge auf jedem erfassten Bild durch eine einzige und jeweils unterschiedliche Lichtquelle punktuell beleuchtet. Das abgebildete Auge, und die darin sichtbaren Reflexe, können somit eindeutig einem Lichteintrittswinkel zugeordnet werden. Für jedes der abgespeicherten Bilder wird vorzugsweise zusätzlich eine dazugehörige Information über die während der Bildgebung angeschaltete Lichtquelle gespeichert.

Vorzugsweise sind die Lichtquellen um die Kameralinse entlang wenigstens einem Kreisteil angeordnet. Zum Beispiel können die Lichtquellen an einer schräg zur Kameraachse orientierten Ringfläche oder Teilringfläche einer Trägerplatte angeordnet sein. Die Anordnung ist hierbei so zu wählen, dass eine gleichbleibende Helligkeit der entstehenden Purkinje-Reflexe gewährleistet wird. Hierzu ist zu beachten, dass das Licht aus der jeweiligen Richtung senkrecht zur Hornhautsoberfläche in das Auge einfällt. Hierzu besitzt die Trägerplatte vorzugsweise eine entsprechende konvexe Krümmung. Eine gleichbleibende Helligkeit verschiedener Bilder der Bildfolge erleichtert die automatische Erkennung der Purkinje-Reflexe und mach eine Post-Prozessierung der Bilder im besten Fall überflüssig.

Die Trägerplatte, auf welcher die Infrarot-LEDs angeordnet sind, umfasst in einem bevorzugten Ausführungsbeispiel zusätzlich einen einzigen visuell erkennbaren Referenzpunkt. Dieser Referenzpunkt wird während der Bilderfassung kontinuierlich vom abzubildenden Auge fokussiert, während in vorbestimmter Reihenfolge Licht der Infrarot-LEDs aus unterschiedlichen Winkeln in das Auge einfällt. Der Referenzpunkt kann vorzugsweise eine zusätzliche Lichtquelle sein, welche sichtbares Licht abgeben kann, und welche dazu eingerichtet ist, während des Erfassens jedes der Bilder eingeschaltet zu sein

Alternativ dazu kann eine erfindungsgemässe Vorrichtung ausserhalb der Trägerplatte an einer vorbestimmten Position diesen einzigen visuell erkennbaren Referenzpunkt umfassen. Der Referenzpunkt kann eine zusätzliche Lichtquelle sein, welche sichtbares Licht abgeben kann, und welche dazu eingerichtet ist, während des Erfassens jedes der Bilder eingeschaltet zu sein. Das sichtbare Licht, welches von dieser Referenzlichtquelle ausgestrahlt wird, kann vorzugsweise über optische Mittel in das Sichtfeld des abzubildenden Auges eingeleitet werden. Die optischen Mittel umfassen beispielsweise wenigstens einen Spiegel. Das Bereitstellen eines Referenzpunktes ist unabhängig von anderen erfindungsgemässen Merkmalen und kann in allen erfindungsgemässen Ausbildungen bereitgestellt werden.

Figur 3 zeigt ein Beispiel einer ringförmigen Trägerplatte 131, welche anhand einer Stellschraube 133 rund um eine Kameralinse 114 herum angebracht werden kann. Die Platte umfasst die Lichtquellen 130 und insebesondere fünfzehn infrarot LEDs (1-4 und 6-16) und eine grüne LED, welche schraffiert dargestellt ist und als Referenzpunkt dient.

Damit die Pupillenerkennung (Mittelpunkt der Pupille bildet die Referenz für sämtliche Parameter) automatisch erfolgen kann, muss ein ausreichender SNR ("Signal-to-Noise ratio") sichergestellt werden. Hierzu ist eine ausreichend homogene Ausleuchtung des Auges notwendig. Diese ist von den Infrarot-LEDs 130 entkoppelt und erfolgt vorzugsweise durch eine indirekte Beleuchtung des Auges mittels zusätzlicher infrarot LEDs.

Die erfindungsgemäße Vorrichtung umfasst in einem bevorzugten Ausführungsbeispiel zusätzlich Bildbearbeitungsmittel, welche dazu eingerichtet sind auf den Bildern der Bildfolge einzelne abgebildete Lichtreflexe und deren Verschiebung entlang der Bildfolge zu erkennen. Die Bildbearbeitungsmittel sind vorzugsweise zusammen mit den Steuerungsmittel 140 durch einen entsprechend programmierten Computerprozessor umgesetzt.

Dem erfindungsgemäßen automatischen Erkennungsverfahren der Stellung einer intraokularen oder einer natürlichen Linse liegen, gegenüber dem Stand der Technik folgende Erkenntnisse zu Grunde. Einzelbilder sind nicht ausreichend, um die verschiedenen abgebildeten Reflexe den Purkinje-Reflexen zuzuordnen. Erst ein Vergleich verschiedener Bilder einer Bildfolge, die Reflexe aus unterschiedlichen vordefinierten Beleuchtungsrichtungen zeigt, erlaubt eine zuverlässige Zuordnung. Außerdem können "falsche" Reflexe (Streulicht) von Purkinje-Reflexen erst dann zuverlässig unterschieden werden, wenn sich letztere durch Variation der Beleuchtungsrichtung verschieben, während Reflexionen eines unspezifischen Streulichts ortsfest bleiben.

Das erfindungsgemäße Verfahren geht aus von einem, anhand einer, mittels der erfindungsgemäßen Vorrichtung erfassten Bildfolge, welche das, einen fixen Referenzpunkt fokussierende Auge, abbildet. In einem ersten Schritt werden einzelne abgebildete Lichtreflexe und deren Verschiebung entlang der Bildfolge erkannt oder identifiziert.

Mittels der erkannten Form und der Verschiebung der entsprechenden Lichtreflexe, und mittels des Wissens aus welchem Winkel das Auge in jedem Bild der Bildfolge ausgelichtet wurde, werden verschiedene Lichtreflexe den Purkinje-Reflexen zugeordnet.

Anhand der an sich bekannten Rechenmethoden wird dann die Dezentrierung und/oder Verkippung der natürlichen oder intraokularen Linse anhand der zugeordneten Purkinje-Reflexe berechnet.

Eine automatische Erkennung der abgebildeten Pupillenfläche, welche zum Beispiel teilweise durch ein Augenlid abgedeckt sein kann, kann dazu genutzt werden, die Auswahl der zum Ausleuchten des Auges genutzten Lichtquellen 130 anzupassen, so dass die abgebildeten Reflexe mit hoher Wahrscheinlichkeit auswertbar bleiben.

Anhand der erfindungsgemäßen Maßnahmen kann eine Messung innerhalb weniger Sekunden abgeschlossen werden, innerhalb der der Patient lediglich für den Bruchteil einer Sekunde einen einzigen Punkt fokussieren muss. Die Fehleranfälligkeit des Verfahrens ist somit minimal.

Im Folgenden wird ein bevorzugtes Ausführungsbeispiel des Purkinje-Reflex Erkennungsverfahrens gemäss der vorliegenden Erfindung erläutert.

### Bilderfassung

Anhand einer bevorzugten Ausführungsform der erfindungsgemäßen Vorrichtung wird das Auge während dem Erfassen der Bildfolge mittels des in Figur 3 dargestellten LED-Rings 131 ausgeleuchtet. Die Distanz zwischen der Kameralinse und dem zu erfassenden Auge beträgt ungefähr 35 cm. Die gesamte Erfassungsprozedur ergibt sechzehn grauton 8-Bit-Bilder mit einer Bildgröße von 640 x 480 Pixel, entsprechend 15 Infrarot-LEDs, in denen in jedem Bild nur eine infrarot LED angeschaltet ist, und ein zusätzliches dunkles Bild, bei dem alle infrarot LEDs ausgeschaltet sind.

### Bildanalyse

Der Algorithmus für die automatische Erkennung der Purkinje-Reflexe besteht aus drei aufeinanderfolgenden Schritten: Pupillenerkennung, Partikelerkennung (Reflexerkennung) und Partikelanalyse (Reflexanalyse). Zunächst muss die Pupille erkannt und zusammen mit ihren Mittenkoordinaten in jedem Bild extrahiert werden. Diese Informationen können verwendet werden, um die Bilder auf den Pupillenbereich zu reduzieren. Im nächsten Schritt wird nur der Pupillenbereich zum Suchen der Purkinje-Reflexe betrachtet. Neben den Purkinje-Reflexe kann das Bild Artefakte in Form von hellen Flecken enthalten. Alle beleuchteten Punkte werden wie eine Wolke von Teilchen behandelt. Nach Detektion der Partikel werden deren Koordinaten analysiert. Die Purkinje-Reflexe P1, P3 und P4 werden dann erkannt. Der nächste Schritt beinhaltet den Berechnungsvorgang, bei dem die Koordinaten der Purkinje-Reflexe zum berechnen der Position und Orientierung der natürlichen oder intraokularen Linse verwendet werden.

### Pupillenerkennung

Die Pupillenerkennungsalgorithmus nutzt eine einfache Schwellentechnik auf der Grundlage von Bild-Histogrammen, welche die Anzahl der Pixel für jeden Grauwert anzeigen. Obwohl sie in Proportion und Größe variieren, können einige Bildmerkmale in allen Bildern der erfassten Bildfolge, die das ordnungsgemäß beleuchtete Auge abbilden, gefunden werden. Der Abschnitt der Graustufenpixel, der die Iris und die Augenlider umfasst, liegt typischerweise im Bereich von bis zu 100. Diese Pixel werden aus dem Bild extrahiert, und der Rand der Pupille unter Verwendung eines Canny-Filter erfasst. Die tatsächlichen Werte hängen selbstverständlich von der erhaltenen Helligkeit der Bilder ab.

Basierend auf diesen Informationen, lässt sich die Pupille am geeignetsten anhand einer gedrehten Ellipse beschreiben. Parameter der Ellipse wie deren Mitte und Rand können mathematisch auf bekannte Weise ausgedrückt werden.

### Reflexerkennung

Die zweite Stufe der Bildverarbeitung umfasst die Detektion der Purkinje-Reflexe in jedem der von der erfindungsgemäßen Vorrichtung erfassten Bilder. Die Purkinje-Reflexe P1, P3 und P4 verfügen in der Regel über charakteristische Eigenschaften, welche für deren Erkennung hilfreich sind. P1 kann häufig leicht durch seine sternförmige Erscheinung erkannt werden, häufig erscheint P3 größer als P1 und diffuser. P4 in den meisten Fällen annähernd kreisförmig und beträchtlich kleiner als die anderen beiden Reflexe. In Bildern von pseudophaken, das heißt mit intraokularen Linsen implantieren Augen werden oft die drei Reflexe mit ähnlicher Helligkeit beobachtet.

Häufig werden zusätzlichen Teilchen oder Reflexe in der Bildfolge erfasst, so dass zunächst alle Teilchen innerhalb des Pupillenbereichs erkannt werden müssen. Hauptgründe dafür sind Reflexionen, die durch nicht-ideale Lichtbedingungen im Untersuchungsraum sowie helle Bereiche der Iris und anderer Bereiche des Auges am Rande der Ellipse hervorgerufen werden.

Es wird davon ausgegangen, dass die Purkinje-Reflexe die hellsten Pixel im Pupillenbereich umfassen. Das Entfernen isolierter heller Pixel kann optional in einem nachfolgenden Schritt erforderlich sein. Der letzte Teil dieses Schritts erfordert die Anwendung eines an sich bekannten Verfahres zum Ermitteln der Koordinaten eines jeden Reflexes durch alle Bilder.

### Zuweisen von Reflexen zu entsprechenden Purkinie-Reflexen

Wie bereits beschrieben, verschiebt sich die Position der Purkinje-Reflexe mit jeder einzelnen Infrarot-LED-Beleuchtung. Die Bewegung der Purkinje-Reflexe durch die Bilder entspricht den zwei Halbkreisen LED auf dem LED-Ring.

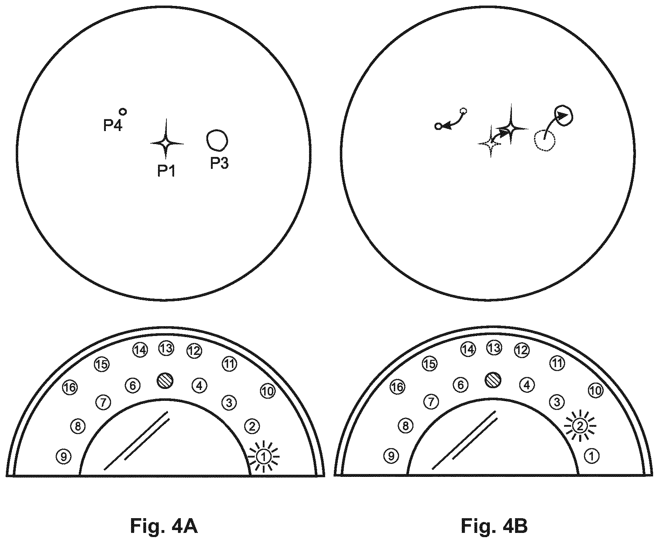

In der schematischen Darstellung des Pupillenbereichs in Figuren 4A und 4B wird gezeigt, dass P4 sich in Richtung des linken Pupillenrands bewegt, wenn die LEDs 1 und 2 nacheinander wie abgebildet eingeschaltet werden. P4 Reflexe, welche mittels einer Beleuchtung aus dem unteren Halbkreis des LED-Rings erzeugt werden, erscheinen unterhalb der Orte der Reflexe, welche aus dem oberen Halbkreis resultieren.

Bezüglich der Lage von P1 und P3 kann das Gegenteil zu beobachtet werden. Sie bewegen sich in Richtung des rechten Pupillenrands. Reflexe, die aus der Beleuchtung einer Lichtquelle der unteren LED-Reihe resultieren, erscheinen oberhalb der entsprechenden Reflexe, welche aus einer Beleuchtung aus der oberen Reihe erfolgen. Es ist wichtig zu beachten, dass die exakten Standorte und die Reihenfolge der Reflexe, die in dieser schematischen Darstellung zu sehen sind, stark von den Eigenschaften des gemessenen Auges abhängig sind.

Das Verfolgen der Reflexe beginnt im ersten Bild. Für jedes der darauf folgenden Bilder wird Bezug auf die respektive Position von P1, P3 und P4 im vorhergehenden Bild genommen. Insbesondere wird die unmittelbaren Nähe der Position der drei Punkte im vorhergehenden Bild analysiert.

Zunächst wird der erste erkannte Reflex des zur LED 1 zugeordneten Bilds als ursprüngliches Teilchen betrachtet. In dem nächsten Bild wird in einem Umfang rund um die Position des ursprünglichen Teilchens nach ähnlich grossen Teilchen gesucht. Falls eines oder mehrere solcher Teilchen innerhalb des Suchumfangs gefunden werden, werden diese als ursprüngliche Teilchen in der folgenden Iteration genutzt. Falls keine Teilchen gefunden werden, werden die Partikel des gesamten nächsten Bildes, deren Größe in der Nähe der Größe des ursprünglichen Teilchens liegt, als die nächsten ursprünglichen Teilchen ausgewählt. Dieses Verfahren wird wiederholt, bis das Bild der LED # 16 erreicht ist. Nachdem dieser Zyklus für die ersten Teilchen in dem ersten Bild beendet ist, wird der gesamte Vorgang für alle verbleibenden Teilchen in dem der LED 1 zugeordneten Bild wiederholt. Am Ende hat man für jedes Teilchen in dem der LED zugeordnetem Bild eine Menge oder Gruppe an entsprechenden Teilchen in den folgenden Bildern identifiziert.

Wie in Figur 3 gezeigt, kann eine Lücke zwischen LED # 4 und LED # 6 in der Anordnung der LEDs auf dem LED-Ring beobachtet werden. In einem solchen Fall kann der Suchumfang für die Teilchen der entsprechenden Bildern entsprechend vergrößert werden.

In einem folgenden Schritt wird entschieden, welche Teilchen einer Purkinje-Reflexion entsprechen. Das Verfahren unterscheidet zwischen P1, P3 und P4 unter Berücksichtigung ihrer besonderen Eigenschaften, wie Größe, Richtung und der Entfernungen, die sie von einem Bild zum nächsten zurücklegen.

P4 beispielsweise bewegt sich gegen den Uhrzeigersinn und hat in der Regel entlang der Bildfolge eine ähnliche Größe. Deshalb dienen zum Erkennen des P4 Reflexes von einem Bild zum anderen jene Teilchen, deren Größe sich am wenigsten verändert. Dieses Verfahren wird für jede der vorher identifizierten Gruppen von Teilchen wiederholt, um P4 mit hoher Wahrscheinlichkeit zu erkennen.

Ähnlich zu dem vorhergehenden Schritt zur Identifikation des P4 Reflexes werden charakteristische Merkmale der Purkinje-Reflexe P1 und P3 verwendet um diese zu erkennen. Während beide Reflexe sich in Richtung des rechten Pupillenrands bewegen, sind die Verschiebungsabstände von einem Bild zum nächsten unterschiedlich. Normalerweise decken P3 Reflexe größere Entfernungen im Vergleich zu P1 ab. Die Berechnung der entsprechenden Abstände für jedes Bild ergibt dann eine robuste Methode, um die Reflexe in einem letzten Schritt zu ermitteln.

### Bezugszeichenliste

- 1: Hornhaut
- 2: Linse
- 100: Vorrichtung
- 102: Bildfolge
- 110: Kamera
- 111: Kameraachse
- 112: bildgebende Mittel
- 114: Kameralinse
- 120: Stellungsmittel
- 130: Lichtquellen
- 131: Trägerplatte
- 140: Steuerungsmittel
- 150: Speichermedium

## Patentansprüche

1. Vorrichtung (100) zum automatischen Erkennen der Stellung einer intraokularen Linse oder einer natürlichen Linse in einem Auge, umfassend:
- eine Kamera (110), welche bildgebende Mittel (112) und eine Kameralinse (114) umfasst, und eine Kameraachse (111) definiert;
- Stellungsmittel (120), die dazu ausgebildet sind die Kamera (110) gegenüber dem abzubildenden Auge in eine fixe, vorbestimmte Stellung zu bringen;
- eine Vielzahl an einzelnen Lichtquellen (130), welche so angeordnet sind, dass jede der Lichtquellen (130) Lichtstrahlen (132) in einem jeweils vorbestimmten Winkel gegenüber der Kameraachse (111) und in Richtung des abzubildenden Auges abgegeben kann;
- Steuerungsmittel (140), die dazu ausgebildet sind mittels der Kamera (110), bei gleichbleibender Stellung der Kamera (110) und der Lichtquellen (130), eine Bildfolge (102) des abzubildenden Auges zu erfassen und auf einem Speichermedium (150) zu sichern,
**dadurch gekennzeichnet, dass**
die Steuerungsmittel (140) des Weiteren dazu ausgebildet sind, die Lichtquellen (130) so ein- und auszuschalten, dass während des Erfassens der Bilder die Lichtquellen in einer vorbestimmten Reihenfolge eingeschaltet werden, und dass während des Erfassens jedes der Bilder jeweils nur eine vorbestimmte Lichtquelle (130) eingeschaltet ist; und die Vorrichtung Bearbeitungsmittel umfasst, welche dazu eingerichtet sind, auf den Bildern der Bildfolge einzelne abgebildete Lichtreflexe und deren Verschiebung entlang der Bildfolge zu erkennen, aufgrund der erkannten Form und Verschiebung der entsprechenden Lichtreflexe spezifische Purkinje-Reflexe zuzuordnen, und aufgrund der zugeordneten spezifischen Purkinje-Reflexe auf die Stellung der intraokularen oder der natürlichen Linse in dem abgebildeten Auge zu schließen.

2. Vorrichtung gemäß Anspruch 1, wobei die Lichtquellen um die Kameralinse entlang wenigstens einem Kreisteil angeordnet sind.

3. Vorrichtung gemäß einem der Ansprüche 1 oder 2, wobei die Lichtquellen an einer schräg zur Kameraachse orientierten Ringfläche oder Teilringfläche angeordnet sind.

4. Vorrichtung gemäß einem der Ansprüche 1 bis 3, wobei die Lichtquellen Infrarot-Leuchtdioden sind und wobei die bildgebenden Mittel infrarot-empfindliche bildgebende Mittel sind.

5. Vorrichtung gemäß einem der Ansprüche 1 bis 4, wobei die Lichtquellen auf einer Trägerplatte angeordnet sind.

6. Vorrichtung gemäß Anspruch 5, wobei die Trägerplatte einen einzigen visuell erkennbaren Referenzpunkt umfasst.

7. Vorrichtung gemäß Anspruch 6, wobei die Trägerplatte eine zusätzliche Lichtquelle umfasst, welche sichtbares Licht abgegeben kann, und welche dazu eingerichtet ist während des Erfassens jedes der Bilder eingeschaltet zu sein.

8. Verfahren zum automatischen Erkennen der Stellung einer intraokularen Linse oder einer natürlichen Linse in einem Auge anhand einer mittels der Vorrichtung gemäß einem der Ansprüche 1 bis 7 erfassten Bildfolge welche das Auge abbildet, umfassend:
- Erkennen von einzelnen abgebildeten Lichtreflexen und deren Verschiebung entlang der Bildfolge;
- Zuordnen der Lichtreflexe zu Purkinje-Reflexe mittels der erkannten Form und der Verschiebung der entsprechenden Lichtreflexe sowie des vorbestimmten Winkels der jeweilig entsprechenden Lichtquelle (130), welche während des Erfassens eines jeweiligen Bildes eingeschaltet war;
- Errechnen der Dezentrierung und/oder Verkippung der Linse anhand der zugeordneten Purkinje-Reflexe.

9. Verfahren gemäß Anspruch 8, worin des Weiteren ein Umriss der Pupille und/oder der Iris des abgebildeten Auges in der Bildfolge automatisch erkannt wird.

## Claims

1. A device (100) for automatically detecting the position of an intraocular lens or a natural lens in an eye, comprising:
- a camera (110) comprising imaging means (112) and a camera lens (114) and a camera axis (111);
- positioning means (120) adapted to bring the camera (110) relative to the eye to be imaged in a fixed, predetermined position;
- a plurality of individual light sources (130), which are arranged so that each of the light sources (130) can emit light beams (132) at a respective predetermined angle with respect to the camera axis (111) and in the direction of the eye to be imaged;
- control means (140) which are configured, by means of the camera (110) with a constant position of the camera (110) and the light sources (130), to capture an image sequence (102) of the eye to be imaged and to secure said sequence on a storage medium (150), **characterized in that** the control means (140) are further configured to switch the light sources (130) on and off so that during capture of the images, the light sources are turned on in a predetermined order and that during the capture of each of the images only one predetermined light source (130) is turned on; and the device additionally comprises processing means which are adapted to recognize on the images of the image sequence individual imaged light reflections, and their displacement along the image sequence, to assign Purkinje-reflexes by means of the recognized shape and the displacement of the corresponding light reflections, and to determine the position of an intraocular lens or a natural lens in an eye based on the assigned Purkinje-reflexes.

2. Device according to claim 1, wherein the light sources are arranged around the camera lens along at least one arc of circle.

3. Device according to one of claims 1 or 2, wherein the light sources are arranged on an annular surface, or partial annular surface, obliquely oriented to the camera axis.

4. Device according to any of claims 1 to 3, wherein the light sources are infrared light emitting diodes and wherein the imaging means are infrared sensitive imaging means.

5. Device according to one of claims 1 to 4, wherein the light sources are arranged on a support plate.

6. The device according to claim 5, wherein the carrier plate comprises a single visually recognizable reference point.

7. Apparatus according to claim 6, wherein the support plate comprises an additional light source capable of emitting visible light and which is arranged to be turned on during the detection of each of the images.

8. A method for automatically detecting the position of an intraocular lens or a natural lens in an eye on the basis of an image sequence acquired by the device according to any one of claims 1 to 7, which images the eye, comprising:
- recognition of individual imaged light reflections and their displacement along the image sequence;
- assigning of the light reflections to Purkinje-reflexes by means of the recognized shape and the displacement of the corresponding light reflections and the predetermined angle of the respectively corresponding light source (130), which was switched on during the capture of a respective image;
- calculating the decentration and/or tilt of the lens based on the assigned Purkinje reflexes.

9. The method of claim 8, further comprising automatically recognizing an outline of the pupil and/or iris of the imaged eye in the image sequence.

## Revendications

1. Dispositif (100) de détection automatique de la position d'un cristallin intraoculaire ou d'un cristallin naturel dans un oeil, comprenant :
- une caméra (110) comprenant des moyens d'imagerie (112) et une lentille de caméra (114), et définissant un axe de caméra (111) ;
- des moyens de positionnement (120), qui sont conçus pour amener la caméra (110) dans une position fixe et prédéterminée par rapport à l'oeil à imager ;
- une pluralité de sources lumineuses individuelles (130), qui sont agencées de telle sorte que chacune des sources lumineuses (130) peut émettre des rayons lumineux (132) selon un angle respectif prédéterminé par rapport à l'axe de la caméra (111) et en direction de l'œil à imager ;
- des moyens de commande (140), qui sont conçus pour saisir, au moyen de la caméra (110), une séquence d'images (102) de l'œil à imager et pour la sauvegarder sur un support de stockage (150), la position de la caméra (110) et des sources lumineuses (130) restant constante,
**caractérisé en ce que** les moyens de commande (140) sont en outre conçus pour allumer et éteindre les sources lumineuses (130) de telle sorte que, pendant la saisie des images, les sources lumineuses sont allumées dans un ordre prédéterminé, et que, pendant la saisie de chacune des images, seule une source lumineuse prédéterminée (130) est allumée à la fois ; et le dispositif comprend des moyens de traitement qui sont conçus pour reconnaître, sur les images de la séquence d'images, des réflexions lumineuses individuelles représentées et leur déplacement le long de la séquence d'images, pour attribuer des réflexions de Purkinje spécifiques sur la base de la forme et du déplacement reconnus des réflexions lumineuses correspondantes, et pour déduire, sur la base des réflexions de Purkinje spécifiques attribués, la position du cristallin intraoculaire ou du cristallin naturel dans l'œil imagé.

2. Dispositif selon la revendication 1, dans lequel les sources lumineuses sont disposées autour de la lentille de la caméra le long d'au moins une partie du cercle.

3. Dispositif selon l'une des revendications 1 ou 2, dans lequel les sources lumineuses sont disposées sur une surface annulaire ou une surface annulaire partielle orientée obliquement par rapport à l'axe de la caméra.

4. Dispositif selon l'une des revendications 1 à 3, dans lequel les sources lumineuses sont des diodes électroluminescentes infrarouges et dans lequel les moyens d'imagerie sont des moyens d'imagerie sensibles à l'infrarouge.

5. Dispositif selon l'une des revendications 1 à 4, dans lequel les sources lumineuses sont disposées sur une plaque de support.

6. Dispositif selon la revendication 5, dans lequel la plaque de support comprend un seul point de référence visuellement détectable.

7. Dispositif selon la revendication 6, dans lequel la plaque de support comprend une source lumineuse supplémentaire capable d'émettre de la lumière visible et qui est conçue pour être allumée pendant l'acquisition de chacune des images.

8. Procédé de détection automatique de la position d'un cristallin intraoculaire ou d'un cristallin naturel dans un oeil à l'aide d'une séquence d'images représentant l'œil acquise au moyen du dispositif selon l'une des revendications 1 à 7, le procédé comprenant les étapes consistant à :
- détecter des réflexions lumineuses individuelles représentées et leur déplacement le long de la séquence d'images ;
- associer les réflexions lumineuses à des réflexions de Purkinje au moyen de la forme détectée et du déplacement des réflexions lumineuses correspondantes ainsi que de l'angle prédéterminé de la source lumineuse correspondante respective (130) allumée pendant l'acquisition d'une image respective ;
- calculer le décentrage et/ou l'inclinaison du cristallin à l'aide des réflexions de Purkinje associées.

9. Procédé selon la revendication 8, dans lequel, en outre, un contour de la pupille et/ou de l'iris de l'œil imagé est détecté automatiquement dans la séquence d'images.
